(19) Europäisches Patentamt
European Patent Office
Office européen des brevets

(11) Veröffentlichungsnummer: **0 047 948**
**B1**

(12) # EUROPÄISCHE PATENTSCHRIFT

(45) Veröffentlichungstag der Patentschrift: **21.09.83**

(21) Anmeldenummer: **81107000.2**

(22) Anmeldetag: **07.09.81**

(51) Int. Cl.³: **C 07 C 141/04,** C 07 C 59/21, C 07 C 69/716, C 07 C 49/16, C 07 C 67/317, C 07 C 51/62, C 07 C 45/51, C 25 B 3/02

(54) **Verfahren zur Herstellung von Polyfluorcarbonylverbindungen sowie einige neue Vertreter dieser Verbindungsklasse.**

(30) Priorität: **12.09.80 DE 3034491**

(43) Veröffentlichungstag der Anmeldung: **24.03.82 Patentblatt 82/12**

(45) Bekanntmachung des Hinweises auf die Patenterteilung: **21.09.83 Patentblatt 83/38**

(84) Benannte Vertragsstaaten: **BE CH DE FR GB IT LI NL**

(56) Entgegenhaltungen:
**DE-A-2 006 094**
**DE-B-2 310 426**
**US-A-3 847 978**

(73) Patentinhaber: **HOECHST AKTIENGESELLSCHAFT, Postfach 80 03 20, D-6230 Frankfurt am Main 80 (DE)**

(72) Erfinder: **Millauer, Hans, Dr., An den sieben Bäumen 21b, D-6236 Eschborn (DE)**
Erfinder: **Siegemund, Günter, Dr., Frankfurter Strasse 21, D-6238 Hofheim am Taunus (DE)**
Erfinder: **Schwertfeger, Werner, Dr., Erlenstrasse 8, D-6306 Langgöns (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Verfahren zur Herstellung von Polyfluorcarbonylverbindungen sowie einige neue Vertreter dieser Verbindungsklasse

Polyfluorierte organische Verbindungen sind Vor-, Zwischen- und Endprodukte auf verschiedenen Sachgebieten. So sind z.B. perfluorierte Ketocarbonsäureester wie die Ester der Heptafluorisopropylglyoxylsäure $(CF_3)_2CFCOCOOH$ u.a. wertvolle Wärmeträgerflüssigkeiten und oberflächenaktive Mittel, die sich durch hohe chemische und thermische Stabilität auszeichnen (JP-OS Sho-54-163521).

Bisher wurden fluorierte Ketocarbonsäureester im allgemeinen ausgehend von Dicarbonylverbindungen hergestellt. So beschreibt Zh. Org. Khim 13, 990 (1977) die Umsetzung von Perfluordicarbonsäurefluoriden mit Perfluorolefinen:

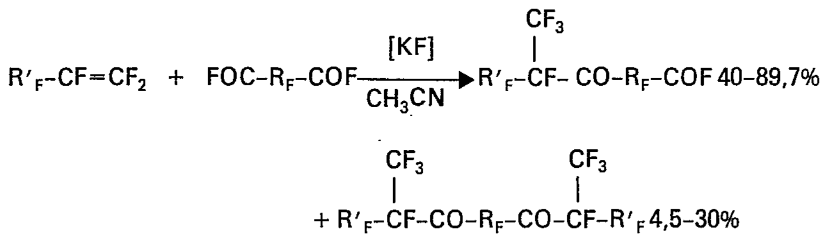


$[R_F = -(CF_2)_4-,\ CF(CF_3)OCF_2-CF_2-,\ -CF(CF_3)O(CF_2)_2O(CF_3)CF-,\ R'_F = CF_3O-, -CF_3]$

Dieses Verfahren hat allerdings den Nachteil, dass neben dem gewünschten Ketocarbonsäurefluorid, das z.B. nach Zh. Org. Khim 11, 1626 (1975) verestert werden kann:

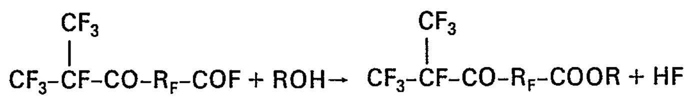

(R = Alkyl)

noch das Diketon mit bis zu 30% Ausbeute anfällt. Analog sind Perfluorketocarbonylverbindungen nach Zh. Org. Khim 11 1626 (1975) ausgehend von Hexafluorpropen und einem Perfluordicarbonsäurefluorid mit nachfolgender Veresterung darstellbar. Auch hier fällt das unerwünschte Diketon in Ausbeuten bis zu 20% an.

Nach den bisher erwähnten Methoden sind keine Ketocarbonsäureester darstellbar, die eine $CF_3$-CO-Gruppe enthalten. Trifluormethylketone mit einer Estergruppe sind bisher nur nach zwei speziellen Methoden erhältlich.

In Dokl. Akad. Nauk SSSR 196, 594 (1966) ist die Herstellung von 2-Oxotrifluorpropansäureestern beschrieben.

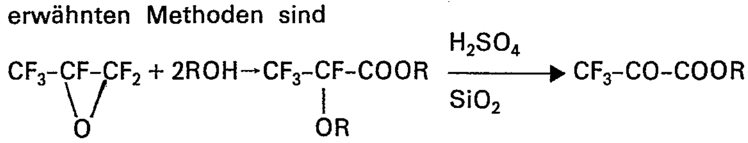


(R = Alkyl)

Diese Reaktion kann, weil sie von einem Epoxid ausgeht, nur vicinale Ketocarbonsäureester liefern.

In J. Am. Chem. Soc. 75 3152 (1953) ist die Herstellung von 3-Oxoperfluorbutansäureestern beschrieben. Die Reaktion erfolgt nach der Gleichung:

$$NaH + H-CF_2-COOR \xrightarrow[-H_2]{} NaCF_2-COOR \xrightarrow[-NaOR]{+CF_3-COOR} CF_3-CO-CF_2-COOR$$

Der Butylester ($R = C_4H_9$) soll nur in Form des Hemiketals (mit Butanol) isoliert und der Ethylester soll in ca. 20%iger Ausbeute erhalten worden sein. Nach eigenen Versuchen kann es sich bei letzterer Verbindung jedoch nicht um den Ethylester der 3-Oxo-perfluorbuttersäure gehandelt haben (vgl. nachstehendes Beispiel 13).

Die beiden zuletzt genannten Verfahren sind jeweils nur für die Herstellung von 2- bzw. 3-Oxoperfluorcarbonsäureestern anwendbar.

Aufgabe dieser Erfindung war es nun, ein einfaches Verfahren zur Herstellung sowohl bekannter als auch neuer Perfluorcarbonylverbindungen, insbesondere von Perfluorketocarbonsäureestern, Perfluorketocarbonsäurehalogeniden und Perfluordiketonen, letztere möglichst mit

zwei verschieden substituierten Ketogruppen, zu finden.

Das Verfahren sollte allgemein anwendbar sein und die gewünschten Verbindungen in hoher Ausbeute und Reinheit liefern.

Die gestellte Aufgabe konnte erfindungsgemäss dadurch gelöst werden, dass man

a) Perfluorcarbonylverbindungen mit noch einem sec.-H-Atom der Formel I

$$\begin{array}{l} \qquad\qquad\qquad\quad CF_3 \\ \qquad\qquad\qquad\quad | \\ R_f\text{-}CF\text{-}R_f'\text{-}[CF_2\text{-}O\text{-}CF\text{-}]_n\text{-}COR'' \\ \quad | \\ \quad H \end{array} \qquad (I),$$

worin $R_f$ = Perfluoralkyl mit 1–10, vorzugsweise 1–8, insbesondere 1–3 C-Atomen,

$R_f'$ = Perfluoralkylen mit 1–10, vorzugsweise 1–6, insbesondere 1–2 C-Atomen,

$R''$ = F, Cl oder

Perfluoralkyl mit 1–10, vorzugsweise 1–8, insbesondere 1–3 C-Atomen, und

n = 0–10, vorzugsweise 0–4, insbesondere 0 oder 1

bedeuten,

in einem Elektrolyten aus Fluorsulfonsäure und einem Alkalifluorsulfonat unter Verwendung von Platin oder Metallen der Platingruppe (Os, Ir, Pt) und/oder glasartigem Kohlenstoff als Anodenmaterialien und von üblichen, jedoch unter den Elektrolysebedingungen stabilen Kathodenmaterialien zu den Fluorsulfatoverbindungen der Formel II

$$\begin{array}{l} R_f\text{-}CF\text{-}R'_f\text{-}[CF_2\text{-}O\text{-}CF]_n\text{-}COR'' \\ \quad | \qquad\qquad\qquad\quad | \\ \quad OSO_2F \qquad\qquad CF_3 \end{array} \qquad (II)$$

worin $R_f$, $R'_f$, $R''$ und n die gleiche Bedeutung wie in Formel I besitzen, elektrolysiert,

b) die Fluorsulfatoverbindungen der Formel II gegebenenfalls – wenn $R''$ = F oder Cl – mit einer organischen Hydroxylverbindung der Formel III

$$R'''OH \qquad (III),$$

worin $R'''$ = Alkyl, Aryl oder Aralkyl mit vorzugsweise bis zu 10 C-Atomen, insbesondere $CH_3$ oder $C_2H_5$ bedeutet, verestert, und

c) die nach den Reaktionsstufen a und b resultierenden Verbindungen der Formel IV

$$\begin{array}{l} R_f\text{-}CF\text{-}R'_f\text{-}[CF_2\text{-}O\text{-}CF]_n\text{-}COR \\ \quad | \qquad\qquad\qquad\quad | \\ \quad OSO_2F \qquad\qquad CF_3 \end{array} \qquad (IV)$$

worin $R_f$, $R'_f$ und n die gleiche Bedeutung wie in den Formeln I und II besitzen und R = $R''$ wie in Formel I und II oder $-OR'''$ mit $R'''$ wie in Formel III, in Gegenwart katalytischer Mengen von Alkalifluoriden und/oder aprotischen N-Basen zu Verbindungen der Formel V

$$\begin{array}{l} R_f\text{-}C\text{-}R'_f\text{-}[CF_2\text{-}O\text{-}CF]_n\text{-}COR \\ \quad \| \qquad\qquad\qquad\quad | \\ \quad O \qquad\qquad\qquad CF_3 \end{array} \qquad (V)$$

worin $R_f$, $R'_f$, R und n die gleiche Bedeutung wie in Formel IV besitzen, zersetzt.

Die erste Stufe a) des erfindungsgemässen Verfahrens besteht in der Substitution eines Wasserstoffatoms durch eine Fluorsulfatogruppe. Derartige Substitutionsreaktionen sind bei polyfluorierten Verbindungen mit einem primären Wasserstoffatom ($-CF_2H$) bekannt.

C.G. Krespan beschreibt in Journal of Fluorine Chemistry 2, 173 (1972/73) die Umsetzung von 1-H-Perfluorpropan mit Peroxodisulfuryldifluorid ($FSO_2\text{-}O\text{-}O\text{-}SO_2F$) zu 1-Fluorsulfatoperfluorpropan. Auch aus ω-H-Perfluorcarbonsäurenitrilen entstehen in entsprechender Weise, wenn auch in geringerer Ausbeute, ω-Fluorsulfatoperfluorcarbonsäurenitrile.

Nachdem bereits von F.B. Dudley, J. Chem. Soc. 1963, 3407 gezeigt worden war, dass Peroxodisulfuryldifluorid bei der anodischen Oxidation von Lösungen von Alkalifluorsulfonaten in Fluorsulfonsäure an Platinelektroden gebildet wird, vereinigten A. Germain und A. Commeyras, J. Chem. Soc. Chem. Comm. 1978, 118, beide Schritte zu einem einstufigen elektrochemischen Prozess. Ausgehend von 1-H-Perfluoralkanen erhielten sie dabei durch «anodische Funktionalisierung» in Fluorsulfonsäure/Kaliumfluorsulfonat-Elektrolyten an Platin durch einen indirekt über die Peroxodisulfuryl-difluorid-Zwischenstufe verlaufenden Prozess die entsprechenden 1-Fluorsulfatoperfluoralkane.

Es gelingt nach dem erfindungsgemässen Verfahren nunmehr unerwartet glatt, diese Reaktion auf sekundäre Wasserstoffatome (–CHF–) in den Monohydroperfluorcarbonylverbindungen I zu übertragen, und die bisher unbekannten Fluorsulfatoperfluorcarbonylverbindungen der allgemeinen Formel II darzustellen. Dabei ist es überraschend, dass keine kathodische Reduktion der Carbonylgruppe eintritt.

Die Elektrolyse kann daher in einfachen, ungeteilten Zellen durchgeführt werden, wobei für das erfindungsgemässe Vorgehen normale Labor-Becherglaszellen benutzt werden können, ohne dass die Erfindung darauf beschränkt werden soll.

Als Anode eignen sich Osmium, Iridium, Platin oder Platinlegierungen mit etwa bis zu 10% an anderen Edelmetallen, beispielsweise Iridium. Vorzugsweise besteht die Anode jedoch aus glasartigem Kohlenstoff («Glassy Carbon»), der sich unter den Elektrolysebedingungen als besonders korrosionsbeständig erweist. Glasartiger Kohlenstoff eignet sich auch als Kathode, obwohl die Materialfrage für diese Elektrode nicht kritisch ist und andere Stoffe, wie Platin, Kupfer oder Edelstahl ebenfalls dafür geeignet sind.

Über die Herstellung, Struktur und Eigenschaften von glasartigem Kohlenstoff siehe z.B. den Artikel «Thermischer Abbau von Polymeren bis zum elementarem Kohlenstoff – ein Weg zu

Werkstoffen der Zukunft» von E. Fitzer in Angew. Chem. 92, 375 bis 386 (1980).

Das Flächenverhältnis von Anode zu Kathode liegt zwischen etwa 1:1 bis vorzugsweise etwa 5:1 bis 10:1.

Zur Bereitung des Grundelektrolyten, welcher aus Fluorsulfonsäure und einem darin gelösten Alkalifluorsulfonat besteht, löst man zweckmässig ein entsprechendes leicht zugängliches Alkalichlorid, wie beispielsweise Lithiumchlorid, Natriumchlorid oder Kaliumchlorid, in Fluorsulfonsäure, die man gegebenenfalls vorher einer Reinigung durch fraktionierte Destillation unterzogen hat, wobei sofort die Hauptmenge an Chlorwasserstoff aus der Lösung entweicht. Der Rest wird durch Einleiten von trockenem Stickstoff ausgetrieben. Die anzuwendende Konzentration des Alkalisulfonats im Grundelektrolyten ist nicht kritisch und liegt vorzugsweise im Bereich von etwa 0.05 bis etwa 3 Mol pro Liter. Oxidierbare Verunreinigungen oder Feuchtigkeitsspuren werden gegebenenfalls durch eine Vorelektrolyse beseitigt.

Die als Ausgangsstoffe benötigten Monohydroperfluorcarbonylverbindungen der allgemeinen Formel I werden im Grundelektrolyten gelöst oder dispergiert, wobei Gemische mit bis zu etwa 60 Gew.-% Monohydroperfluorcarbonylverbindung, bezogen auf den Grundelektrolyten, zur Anwendung kommen können.

Die Elektrolyse wird im allgemeinen bei einer anodischen Stromdichte von etwa 10–150 mA · $cm^{-2}$, vorzugsweise etwa 20–80 $mA \cdot cm^{-2}$ und einer Temperatur von etwa 0 bis 100°C, vorzugsweise etwa 20–40°C betrieben.

Die Aufarbeitung der Elektrolysegemische und die Isolierung der Fluorsulfatoperfluorcarbonylverbindungen erfolgt in an sich bekannter Weise. Bei zweiphasigen Reaktionsgemischen ist es zweckmässig, die fluororganische Phase, die nur noch wenig Fluorsulfonsäure enthält, durch Scheiden abzutrennen; andernfalls muss das Elektrolyseprodukt von dem Grundelektrolyten destillativ abgetrennt werden. In beiden Fällen kann die Elektrolytphase bzw. der Destillationssumpf nach Zugabe frischer Fluorsulfonsäure wieder in die Elektrolyse recyclisiert werden.

Die rohen Fluorsulfatoperfluorcarbonylverbindungen II können durch fraktionierte Destillation weiter gereinigt werden.

Die Umsetzung von Säurehalogeniden der allgemeinen Formel II, in der R″ Fluor oder Chlor ist, mit einer organischen Hydroxylverbindung der Formel III führt in hoher Ausbeute zu Fluorsulfatoperfluorcarbonsäureestern der allgemeinen Formel IV mit R = OR‴. Dabei war es überraschend, dass diese Reaktion ohne Angriff an der Fluorsulfatogruppe durchgeführt werden konnte. Die Veresterung kann in Gegenwart oder Abwesenheit eines inerten Lösungsmittels wie z.B. Methylenchlorid durchgeführt werden. Während der Reaktion wird die Innentemperatur des Ansatzes zwischen etwa -80 und +70°C, vorzugsweise zwischen etwa -20 und +40°C, insbesondere zwischen etwa 0 und 20°C gehalten.

Das Carbonsäurehalogenid II (mit R″ = F oder Cl) und die organische Hydroxylverbindung III werden zweckmässig im Molverhältnis etwa 1:1 bis etwa 1:1,5 eingesetzt. Ein höherer Überschuss der Hydroxylverbindung III schadet jedoch nicht. Es ist für die erfindungsgemässe Reaktion praktisch ohne Belang, in welcher Reihenfolge die Reaktionskomponenten zusammengegeben werden. Es ist allerdings von Vorteil, durch gutes Rühren für eine gleichmässige Durchmischung des Ansatzes zu sorgen.

Wird für die Veresterung ein Carbonsäurefluorid II mit R″ = F eingesetzt, so entsteht bei der Reaktion Flusssäure, die Borsilikatglas angreift. In diesem Fall ist es von Vorteil, die Reaktion in einem Gefäss aus flusssäurebeständigem Material durchzuführen.

Nach einer bevorzugten Arbeitsweise wird das Carbonsäurehalogenid in einem inerten Lösungsmittel vorgelegt und unter Kühlung mit einer Lösung der Hydroxylverbindung III in dem gleichen Lösungsmittel versetzt. Durch Waschen mit Wasser wird die entstandene Halogenwasserstoffsäure entfernt. Die organische Phase wird abgetrennt und destilliert.

Erfindungsgemäss werden sodann die Fluorsulfatoperfluorcarbonylverbindungen IV mit R = R″ oder = -OR‴, in Gegenwart eines geeigneten Nucleophils (Alkalifluoride und/oder aprotische N-Basen) als Katalysator zu den Ketocarbonylverbindungen der allgemeinen Formel V umgesetzt.

Aus der Literatur sind Spaltungen von sekundären Fluorosulfaten mit Alkalifluoriden bekannt. In Inorg. Chem. 3, 287 (1964) wird der Versuch beschrieben, 2-Fluorsulfonylperfluorpropansäurefluorid mit Alkalifluoriden zu spalten. Während mit Kaliumfluorid keine Reaktion beobachtet wird, tritt mit Caesiumfluorid vollständige Zerstörung des Moleküls ein:

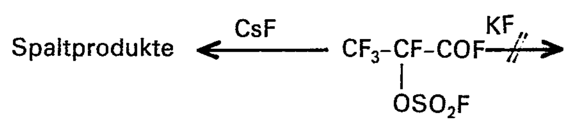


In Inorg. Chem. 4 1441 (1965) wird ein sekundäres Fluorosulfat mit einem etwa 50 molarem Überschuss KF pro Mol Fluorosulfat umgesetzt. Die Umwandlung in das Keton ist erst nach ca. 20 Stunden vollständig (keine Ausbeuteangaben):

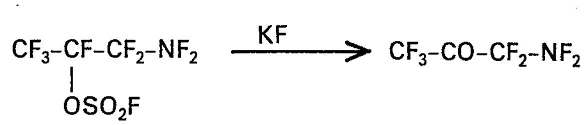


Mit einem grossen Überschuss an Kaliumfluorid wird die in Inorg. Chem. 18 3281 (1979) beschriebene Zersetzung eines sekundären Fluorsulfats durchgeführt. Hier tritt zusätzlich noch eine Umlagerung auf:

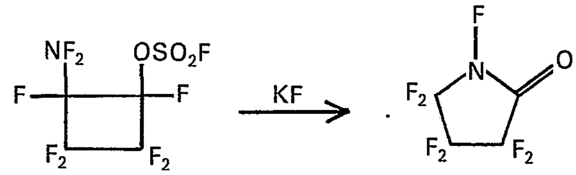


Auch in Inorg. Chem. 5 2184 (1966) ist die Zersetzung von sekundären Fluorosulfaten beschrieben:

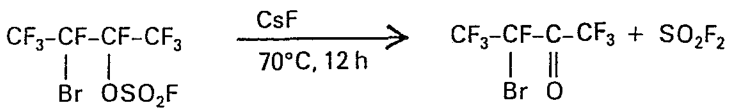


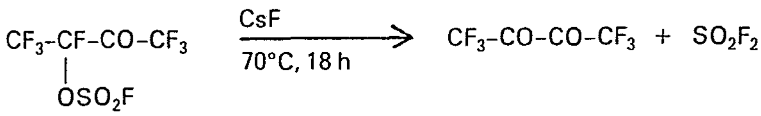


Während im zweiten Fall ein grosser Überschuss an Caesiumfluorid eingesetzt wird, sind im ersten Fall keine Mengenverhältnisse angegeben. Es muss jedoch davon ausgegangen werden, dass wie üblich eine mehr als equimolare Menge des Caesiumfluorids pro Mol Fluorosulfat verwendet wird.

Nach der US-PS 3 549 711 wird 0.17 Mol Fluorsulfatoperfluorcyclobutan mit 0.18 Mol Kaliumfluorid in Gegenwart eines aprotischen, polaren Lösungsmittels zum Perfluorcyclobutanon umgesetzt:

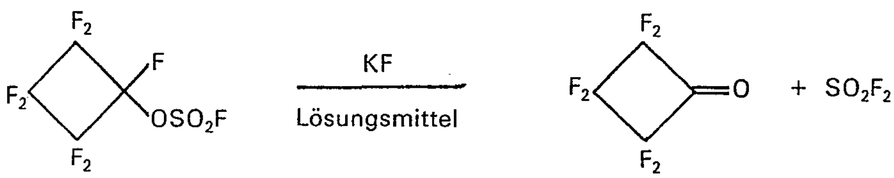


Eine Ausbeuteangabe wird nicht gemacht, da das Keton nicht isoliert, sondern weiter umgesetzt wird.

Reaktionen von sekundären Fluorosulfaten mit tert. Stickstoffbasen sind bisher nicht bekannt.

Es war aufgrund der oben angeführten Beispiele für die Spaltung von sekundären Fluorosulfaten nun überraschend, dass sekundäre Fluorsulfatoperfluorcarbonylverbindungen der Formel IV in Gegenwart von katalytischen Mengen von Alkalifluorid und/oder von aprotischen N-Basen mit hoher Ausbeute in die perfluorierten Ketocarbonylverbindungen V überführt werden können.

Als Katalysatoren können Alkalifluoride und/oder aprotische Stickstoffbasen wie z.B. Triethylamin, 1,4-Diazabicyclo-[2.2.0]octan (DABCO) oder 1.8-Diazabicyclo[5.4.0]undec-7-en (DBU) verwendet werden. Der Katalysator wird vorzugsweise in einer Menge von etwa 1 bis 50 Molprozent, insbesondere von etwa 10 bis etwa 30 Molprozent, bezogen auf die zu zersetzende Verbindung IV, eingesetzt.

Die Reaktionstemperaturen liegen je nach dem verwendeten Katalysator zwischen etwa -40 und +120°C.

Man kann die Reaktion in einem inerten, aprotischen Lösungsmittel durchführen, vorzugsweise wird jedoch ohne Lösungsmittel gearbeitet.

Weiterhin kann sowohl bei Normaldruck als auch unter Überdruck gearbeitet werden.

Für die Reaktionsstufe c) ist es praktisch ohne Belang, in welcher Reihenfolge die Reaktionskomponenten zusammengegeben werden. Es ist allerdings von Vorteil, während der gesamten Dauer der Reaktion für eine gute Durchmischung des Ansatzes zu sorgen.

Nach einer bevorzugten Arbeitsweise gibt man den Katalysator und das Fluorosulfat IV zusammen und erhitzt langsam, bis Gasentwicklung auftritt. Nach beendeter Gasentwicklung wird der Ansatz über eine Kolonne destilliert.

Die nach dem erfindungsgemässen Verfahren hergestellten Ketocarbonylverbindungen V sind farblose und zum Teil feuchtigkeitsempfindliche Flüssigkeiten. Daher ist die Herstellung unter Ausschluss von Feuchtigkeit durchzuführen.

Die Ausgangsverbindungen I für das erfindungsgemässe Verfahren können zum Beispiel nach folgenden bekannten Verfahrensweisen erhalten werden:

a) In J. Am. Chem. Soc. 77, 910 (1955) ist die Herstellung von $CF_3\text{-}CHF\text{-}CF_2\text{-}COCl$ ausgehend vom Natriumsalz der entsprechenden Carbonsäure beschrieben

$$CF_3\text{-}CHF\text{-}CF_2\text{-}COONa \xrightarrow[\text{(Erhitzen)}]{C_6H_5\text{-}COCl} CF_3\text{-}CHF\text{-}CF_2\text{-}COCl$$

b) J. Org. Chem. 42, 4055 (1977) beschreibt unter anderem folgende Reaktionen:

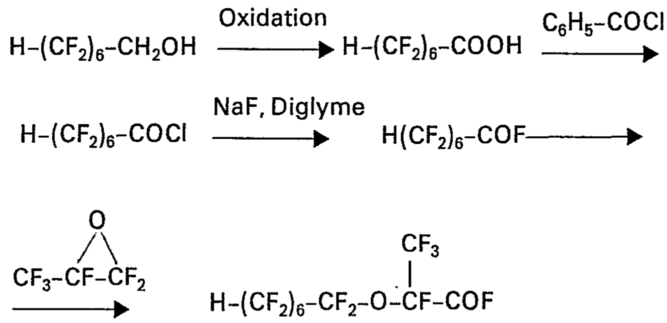


Überträgt man diese Reaktionen auf $CF_3$-CHF-$CF_2$-COCl oder

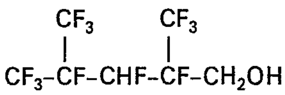

(hergestellt nach Nippon Nagaku Kaishi 1974, 1240) so erhält man z.B. die folgenden Verbindungen, die als Ausgangsmaterial für das erfindungsgemässe Verfahren dienen:

$CF_3$-CHF-$CF_2$-COF

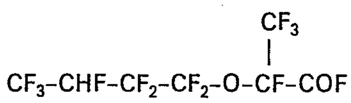

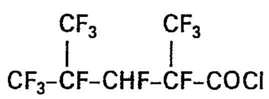

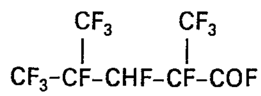

c) In J. Am. Chem. Soc. 84, 4285 (1962) ist die Reaktion von Hexafluorpropen mit Poly- und Perfluorcarbonsäurefluoriden beschrieben:

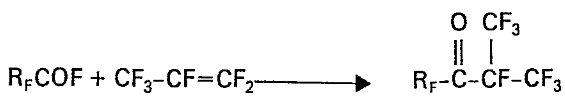

($R_F$ = Perfluoralkyl)

Auch diese Reaktion kann zur Herstellung von Ausgangsverbindungen für das erfindungsgemässe Verfahren verwendet werden:

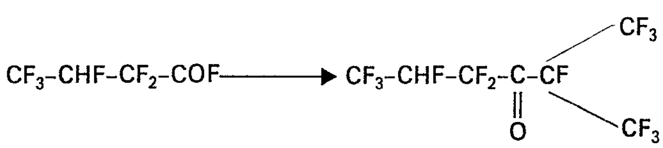

Die nach dem erfindungsgemässen Verfahren hergestellten perfluorierten Ketocarbonsäureester und Diketone, d.s. die Verbindungen der Formel V mit R = OR''' oder Perfluoralkyl sind zum grossen Teil neu und stellen wertvolle Wärmeträgerflüssigkeiten, Schmieröle oder auch Zwischenprodukte für die Herstellung anderer fluororganischer Verbindungen dar. Die Verbindungen der Formel V mit R = F oder Cl sind hauptsächlich Zwischenprodukte für die Herstellung anderer fluororganischer Verbindungen.

Die neuen Verbindungen sind:

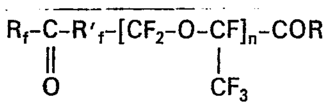 (V)

worin $R_f$ = Perfluoralkyl mit 1-10, vorzugsweise 1-8, insbesondere 1-3 C-Atomen, $R'_f$ = Perfluoralkylen mit 1-10, vorzugsweise 1-6, insbesondere 1-2 C-Atomen, R = F, Cl, Perfluoralkyl mit 1-10, vorzugsweise 1-8, insbesondere 1-3 C-Atomen, oder -OR''' (R''' = Alkyl Aryl oder Aralkyl mit vorzugsweise bis zu 10 C-Atomen, insbesondere $CH_3$ oder $C_2H_5$), und n = 0-10, vorzugsweise 0-4, insbesondere 0 oder 1, mit Ausnahme der Fälle:

a) $R_f$ = $(CF_3)_2CF$-, $R'_f$ = $-(CF_2)_{2-4}-$, R = F oder $OCH_3$ und n = 0,

b) $R_f$ = $(CF_3)_2CF$-, $R'_f$ = $-(CF_2)_5-$, R = F und n = 0, sowie

c) $R_f$= $(CF_3)_2CF$-, $R'_f$ = $-CF_2-$, R = F und n = 1.

Die nachfolgenden Beispiele sollen die Erfindung näher erläutern:

Beispiel 1

Darstellung von 3-Fluorsulfato-perfluorbutansäurefluorid

($CF_3$-CF($OSO_2F$)-$CF_2$-COF)

Die Elektrolysevorrichtung besteht aus einem mit einem äusseren Kühlmantel und einem Deckel versehenen zylindrischen Glasgefäss von etwa 60 mm Innendurchmesser und 100 mm Höhe. Die Zelle ist mit einem als Rückflusskühler wirkenden Trockeneiskühler, ferner mit einem Gaseinleitungsrohr, Thermometer und den Stromzuführungen für die Elektroden versehen. Die Anode besteht aus einer Platte (100 × 20 × 3 mm) aus glasartigem Kohlenstoff, die am Deckel der Zelle befestigt ist und etwa zu ⅔ ihrer Fläche in den Elektrolyt eintaucht. Als Kathode dient ein 1,5 mm starker Platin-Draht, der in einem Abstand von etwa 20 mm parallel zur Anode angeordnet ist.

Als Rührer wird ein PTFE (=Polytetrafluorethylen)-umhüllter Magnetstab auf dem Boden der Zelle benutzt. Die Kühlung der Zelle erfolgt durch einen externen Kühlkreislauf mit Perchlorethylen als Kühlflüssigkeit. Alle medienberührten Teile der Vorrichtung bestehen aus Glas, Platin oder PTFE.

Der Grundelektrolyt wird durch Zugabe von 12,5 g (0,16 Mol) Kaliumchlorid zu 250 g destillierter Fluorsulfonsäure hergestellt; es bildet sich eine farblose Lösung, die durch Einleiten von trockenem Stickstoff von restlichem Chlorwasserstoff befreit und anschliessend 4 Stunden bei einer Stromstärke von 2 A vorelektrolysiert wird.

Nach Zugabe von 190 g (0,96 Mol) 3-Hydro-perfluorbutansäurefluorid wird bei einer Stromstärke von 2–3 A und einer Temperatur von 20°C elektrolysiert, bis ein Ladungsdurchgang von 88 Ah erreicht ist. Die Zellspannung beträgt 6–16 V.

Nach Beendigung der Elektrolyse wird die fluororganische Phase aus dem Elektrolysegemisch abgetrennt und die Elektrolytphase nach Zugabe von 50 g Fluorsulfonsäure mit weiteren 141 g (0,71 Mol) 3-Hydro-perfluorbutansäurefluorid versetzt und unter denselben Bedingungen wie vorher bis zu einer Ladungsmenge von 71 Ah elektrolysiert. Die fluororganische Phase wird erneut durch Scheiden abgetrennt und zusammen mit der 1. Portion fraktioniert destilliert, wobei man 368 g (75% der Theorie) 3-Fluorsulfato-perfluorbutansäurefluorid, Kp. 80–81°C erhält.

$^{19}$F-NMR ($CFCl_3$)*):
+51,52 (1F, $-O-SO_2F$); +25,78 (1F, -CO-F); -77,58 (3F, $-CF_3$); -116,06 (2F, $-CF_2-$); -138,47 (1F, ⪀CF).
*) Für alle $^{19}$F-NMR-Spektren dient $CFCl_3$ als innerer Standard.

Beispiel 2
Darstellung von 3-Fluorsulfato-perfluorbutansäurechlorid
($CF_3-CF(OSO_2F)-CF_2-COCl$)
Unter Benutzung einer Elektrolysevorrichtung wie in dem später folgenden Beispiel 5 beschrieben und nach Bereitung eines Grundelektrolyten aus 250 g Fluorsulfonsäure und 14,6 g (0,25 Mol) Natriumchlorid werden 150 g (0,70 Mol) 3-H-Perfluorbutansäurechlorid bei einer Stromstärke von 2 A und einer Zellspannung von 13–16 V elektrolysiert. Nach 23 Stunden wird die Elektrolyse beendet und das Reaktionsgemisch in einem Scheidetrichter getrennt. Aus der fluororganischen Phase erhält man durch fraktionierte Destillation neben 62 g Ausgangsmaterial 85 g (66% der Theorie, bezogen auf umgesetztes Ausgangsmaterial) 3-Fluorsulfato-perfluorbutansäurechlorid, Kp. 107–108°C.

$^{19}$F-NMR ($CFCl_3$):
+51,12 (1F, $-O-SO_2-F$); -77,50 (3F, $-CF_3-$); -111,55 (2F, $-CF_2$); -137,80 (1F, ⪀CF).

Beispiel 3
Darstellung von 6-Fluorsulfato-perfluor-2-methyl-3-oxaheptansäurefluorid
($CF_3-CF(OSO_2F)-CF_2-CF_2-O-CF(CF_3)-COF$)
Es wird eine Elektrolysevorrichtung, wie in Beispiel 4 beschrieben, verwendet und ein Grundelektrolyt, wie in Beispiel 1 beschrieben, hergestellt.

Nach Zugabe von 100 g (0,27 Mol) 6-Hydro-perfluor-methyl-3-oxa-heptansäurefluorid wird 32 Stunden bei 2 A und 11–13 V Zellspannung elektrolysiert. Die Temperatur beträgt 20°C. Das Elektrolysegemisch wird anschliessend im Scheidetrichter getrennt und die fluororganische Phase (135 g) fraktioniert destilliert. Man erhält neben 17 g Ausgangsmaterial 74 g (70% der Theorie, bezogen auf das umgesetzte Material) 6-Fluorsulfato-perfluor-2-methyl-3-oxa-heptansäurefluorid, Kp. 126–128°C.

$^{19}$F-NMR ($CDCl_3$):
+51,8 (1F, $-O-SO_2F$); +27,4 (1F, -CO-F); -76,63 (0,5 F, d, $J_{gem}$ = 150 Hz, $-O-CF_2-$); -77,27 (3F, $-CF_3$); -77,28 (0,5F, d, $J_{gem}$ = 150 Hz, $-O-CF_2-$); -81,19 (3F, $-CF_3$); -84,06 (0,5F, d, $J_{gem}$ = 150 Hz, $-O-CF_2-$); -84,45 (0,5F, d, $J_{gem}$ = 150 Hz, $-O-CF_2-$); -122,72 (2F, $-CF_2-$); -124,61 (1F, ⪀CF); -137,46 (1F, ⪀CF).

Beispiel 4
Darstellung von 3-Fluorsulfato-perfluor-2,4-dimethyl-pentansäurefluorid
($(CF_3)_2CF-CF(OSO_2F)-CF(CF_3)-COF$)
Es wird eine Elektrolysevorrichtung, wie in Beispiel 1 beschrieben, benutzt, jedoch als Anode eine Platte (100 × 20 × 3 mm) aus galsartigem Kohlenstoff und als Kathode ein Platinstab (Durchmesser 1,5 mm, Länge 100 mm) verwendet.

Nach Bereitung eines Grundelektrolyten aus 250 g Fluorsulfonsäure und 14,6 g (0,25 Mol) Natriumchlorid, wie in Beispiel 1 beschrieben, werden 230 g (0,66 Mol) 3-Hydro-perfluor-2,4-dimethyl-pentansäurefluorid zugefügt und bei einer Stromstärke von 2 A und einer Temperatur von 25°C bis zu einem Ladungsdurchgang von 40 Ah elektrolysiert. Anschliessend wird das Elektrolysegemisch im Scheidetrichter getrennt und die fluororganische Phase (269 g) fraktioniert destilliert. Man erhält dabei neben 53 g Ausgangsmaterial 110 g (48% der Theorie, bezogen auf umgesetztes Material) 3-Fluorsulfato-perfluor-2,4-dimethyl-pentansäurefluorid, Kp. 130°C.

$^{19}$F-NMR ($CDCl_3$):
+54,04 (1F, $-O-SO_2F$); +34,6, +32,31 (1F, -CO-F); -70,73 (9F, breit, $-CF_3$); -121,93, -123,57 (1F, -CF-O-); -173,48, -176,05, -177,69 (2F, ⪀CF).

Beispiel 5

Darstellung von 5-Fluorsulfato-perfluor-2-methyl-hexan-3-on

($(CF_3)_2CF-CO-CF_2-CF(OSO_2F)-CF_3$)

Es wird eine Elektrolysevorrichtung, wie in Beispiel 1 beschrieben, benutzt, jedoch als Kathode ein Stab (Durchmesser 3 mm, Länge 100 mm) aus glasartigem Kohlenstoff verwendet.

Nach Bereitung eines Grundelektrolyten aus 250 g Fluorsulfonsäure und 10,6 g (0,25 Mol) Lithiumchlorid, wie in Beispiel 1 beschrieben, werden 107 g (0,31 Mol) 5-H-Perfluor-2-methyl-hexan-3-on bei einer Stromstärke von 2 A und einer Zellspannung von 12–14 Volt elektrolysiert. Nach 27 Stunden wird die Elektrolyse beendet und das Elektrolysegemisch in einem Scheidetrichter getrennt. Aus der fluororganischen Phase erhält man durch fraktionierte Destillation 101 g (73% der Theorie) 5-Fluorsulfato-perfluor-2-methyl-hexan-3-on, Kp. 109–110°C.

$^{19}F$-NMR ($CDCl_3$):

+51,9 (1F, $-O-SO_2F$); -73,1 (6F, $-CF_3$); -75,9 (3F, $-CF_3$); -114,8 (2F, $-CF_2-$); -136,6 (1F, $-CF(CF_3)_2$); -189,4 (1F, $CF-O-SO_2-F$).

Beispiel 6

Darstellung von 3-Fluor-sulfatoperfluorbutansäuremethylester

$OSO_2F$
|
$CF_3-CF-CF_2-COOCH_3$

Zu einer Lösung von 177,6 g (0,6 Mol) 3-Fluorsulfatoperfluorbutansäurefluorid in 100 ml Methylenchlorid tropft man eine Lösung von 25,6 g (0,8 Mol) Methanol in 30 ml Methylenchlorid. Die Innentemperatur wird während der gesamten Reaktion durch Kühlung unter +10°C gehalten. Der Ansatz rührt eine Stunde bei RT nach und wird dann mehrmals mit Wasser ausgeschüttelt. Nach dem Trocknen der organischen Phase über Natriumsulfat wird über eine Füllkörperkolonne destilliert. Mit Kp. 70°C (60 torr) werden 155 g (84%) 3-Fluorsulfatoperfluorbutansäuremethylester erhalten.

Analyse:

| | | | | |
|---|---|---|---|---|
| Ber. | C 19,49 | H 0,98 | F 43,16 | S 10,41 |
| Gef. | C 19,7 | H 0,9 | F 42,9 | S 10,3 |

$^1H$-NMR ($CDCl_3$): 3,98 (S)
$^{19}F$-NMR ($CDCl_3$): +51,1 ($-OSO_2F$), -77,7 ($CF_3$), -116,4 ($CF_2$), -138,7 (CF)
IR (neat): 5,59 μ (CO), 6,73 μ (SO)

Beispiel 7

Darstellung von 3-Fluorsulfatoperfluorbutansäuremethylester

Die Reaktion wird wie in Beispiel 6 beschrieben durchgeführt. Es werden folgende Mengen eingesetzt: 258 g (0,83 Mol) 3-Fluorsulfatoperfluorbutansäurechlorid, gelöst in 100 ml Methylenchlorid; 38,4 g (1,2 Mol) Methanol, gelöst in 30 ml Methylenchlorid.

Die Destillation liefert 216 g (85%) 3-Fluorsulfatoperfluorbutansäuremethylester.

Beispiel 8

Darstellung von 3-Fluorosulfatoperfluorbutansäureethylester

$OSO_2F$
|
$CF_3-CF-CF_2-COOC_2H_5$

Man legt in einem Kolben 63 g (0,2 Mol) 3-Fluorsulfatoperfluorbutansäurechlorid und 50 ml Methylenchlorid vor. Dann tropft man eine Lösung von 13,8 g (0,3 Mol) Ethanol in 20 ml Methylenchlorid zu. Die Innentemperatur wird dabei zwischen 5 und 10°C gehalten. Der Ansatz rührt eine Stunde bie RT nach und wird anschliessend mehrmals mit Wasser gewaschen und über Natriumsulfat getrocknet. Bei der Destillation werden mit Kp. 152°C (761 mm) 48 g (74%) 3-Fluorsulfatoperfluorbutansäureethylester erhalten.

Analyse:

| | | | | |
|---|---|---|---|---|
| Ber.: | C 22,37 | H 1,56 | F 41,28 | S 9,95 |
| Gef.: | C 22,1 | H 1,5 | F 41,0 | S 9,9 |

$^1H$-NMR ($CDCl_3$): 1,49 (t, J = 7 Hz, 3H, $CH_3$), 4,43 (q, J = 7 Hz, 2H, $CH_2$)
$^{19}F$-NMR ($CDCl_3$): +50,34 ($-OSO_2F$); -78,46 ($CF_3$); -117,35 ($CF_2$); -139,35 (CF)
IR (neat): 5,60 μ (CO), 6,75 μ (SO)

Beispiel 9

6-Fluorsulfatoperfluor-2-methyl-3-oxa-heptansäuremethylester

$CF_3-CF-CF_2-CF_2-O-CF-COOCH_3$
|       |
$OSO_2F$       $CF_3$

99 g (0,21 Mol) 6-Fluorsulfatoperfluor-2-methyl-3-oxa-heptansäurefluorid werden in 80 ml Methylenchlorid gelöst. Zu der auf +5°C abgekühlten Mischung tropft man eine Lösung von 9,6 g (0,3 Mol) Methanol in 30 ml Methylenchlorid. Die Innentemperatur wird während der Reaktion zwischen 5 und 10°C gehalten. Der Ansatz rührt eine Stunde bei RT nach, wird dann mehrmals mit Wasser gewaschen und über Natriumsulfat getrocknet. Die Destillation über eine Füllkörperkolonne ergibt 83 g (84%) Methylester mit Kp. 81–88°C (30 torr) als Diastereomerengemisch (Mengenverhältnis ca. 1:1).

Analyse:

| | | | | |
|---|---|---|---|---|
| Ber.: | C 20,26 | H 0,64 | F 52,09 | S 6,76 |
| Gef.: | C 20,1 | H 0,3 | F 51,8 | S 7,3 |

$^1H$-NMR ($CDCl_3$): 3,96 (S)
$^{19}F$-NMR ($CDCl_3$): +51,7 (1F, $-O-SO_2F$), -76,8 (0,5F, $-CF_2-O-$, $J_{gem}$ = 150 Hz), -77,2 (3F, $CF_3$); -77,7 (0,5F, $-CF_2-O-$, $J_{gem}$ = 150 Hz), -81,6 (3F,

-O-CF($CF_3$)COO); -83,8 (0,5F, -$CF_2$-O-, $J_{gem}$ = 150 Hz); -84,5 (0,5F, -$CF_2$-O-, $J_{gem}$ = 150 Hz); -121,4 ($CF_2$); -131 (-CF-COO-); -137,5 (>CF-O-$SO_2$)
IR (neat): 5,67 µ (C=O), 6,71 µ (S=O)

Beispiel 10
3-Fluorsulfatoperfluor-2,4-dimethylpentansäure-methylester

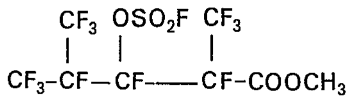

Zu einer Mischung von 30,5 g (0,683 Mol) 3-Fluorsulfatoperfluor-2,4-dimethylpentansäurefluorid mit 30 ml Methylenchlorid tropft man bei 5–10°C Innentemperatur eine Lösung von 3,8 g (0,12 Mol) Methanol in 30 ml Methylenchlorid. Der Ansatz rührt 30 Minuten bei RT nach und wird dann mehrmals mit Wasser gewaschen. Nach dem Trocknen über Natriumsulfat wird über eine Füllkörperkolonne destilliert. Mit Kp. 75–76°C (20 mm) werden 24,7 g (78,7%) 3-Fluorsulfatoperfluor-2,4-dimethylpentansäuremethylester erhalten.

Analyse:

| | | | | |
|---|---|---|---|---|
| Ber.: | C 20,97 | H 0,66 | F 53,91 | S 7,00 |
| Gef.: | C 20,9 | H 0,7 | F 53,9 | S 7,4 |

$^1$H-NMR ($CDCl_3$): 4,03 (S)
$^{19}$F-NMR ($CDCl_3$): +52,45 (1F, -$OSO_2$F); -70,3 bis -72,3 (9F, 3 × $CF_3$); -121,1 und 123,5 (1F, -CF-O-); -174,4; -176,0; -117,9 und -178,8 (2F, CF)
IR (neat): 5,53 µ und 5,60 µ (CO), 6.71 µ (SO)

Beispiel 11
3-Oxo-perfluorbutansäuremethylester
$CF_3$-CO-$CF_2$-$COOCH_3$
Die Reaktion wird in einem Abzug durchgeführt.

In einem trockenen Kolben mit Magnetrührer, Thermometer, Rückflusskühler und nachgeschaltetem Blasenzähler legt man 154 g (0,5 Mol) 3-Fluorsulfatoperfluorbutansäuremethylester und 2,9 g (0,05 Mol) Kaliumfluorid vor. Der Ansatz wird langsam angeheizt. Bei einer Innentemperatur von ca. 60°C beginnt die Abspaltung von Sulfurylfluorid. Nachdem die Gasentwicklung beendet ist, wird der Ansatz über eine Füllkörperkolonne destilliert. Mit Kp. 93°C (755 torr) werden 95,5 g (93%) 3-Oxoperfluorbutansäuremethylester erhalten.

Analyse:

| | | | |
|---|---|---|---|
| Ber.: | C 29,14 | H 1,47 | F 46,10 |
| Gef.: | C 28,80 | H 1,45 | F 46,15 |

$^1$H-NMR ($CDCl_3$): 3,95 (S)
$^{19}$F-NMR ($CDCl_3$): -74,2 ($CF_3$); -112,6 ($CF_2$)
IR (Gasspektrum): 5,35 µ (C=O); 5,55 µ (-COO-)

Beispiel 12
3-Oxoperfluorbutansäuremethylester
Die Reaktion wird in einem Abzug durchgeführt.

In einem trockenen Kolben mit Magnetrührer, Thermometer, Rückflusskühler und nachgeschaltetem Blasenzähler legt man 2,25 g (0,015 Mol) 1,4-Diazabicyclo/2,2,2/octan vor. Dann tropft man 31 g (0,1 Mol) 3-Fluorsulfatoperfluorbutansäuremethylester bei RT zu. Es tritt sofort Gasentwicklung ein. Nachdem alles zugetropft ist, wird die Reaktion durch leichtes Erwärmen vervollständigt. Die anschliessende Destillation liefert 16 g (78%) 3-Oxoperfluorbutansäuremethylester.

Beispiel 13
3-Oxoperfluorbutansäureethylester
$CF_3$-CO-$CF_2$-$COOC_2H_5$
Die Reaktion wird in einem Abzug durchgeführt.

In einem trockenen Kolben mit Magnetrührer, Thermometer, Rückflusskühler und Blasenzähler legt man 45 g (0,139 Mol) 3-Fluorsulfatoperfluorbutansäureethylester und 2,9 g (0,05 Mol) Kaliumfluorid vor. Der Ansatz wird erhitzt bis Gasentwicklung eintritt. Nach dem Ende der Gasentwicklung wird eine Stunde bei 105°C nachgerührt. Die Destillation über eine Füllkörperkolonne lieferte 27 g (88%) 3-Oxoperfluorbutansäureethylester mit Kp 106°C (754 mm).

Analyse:

| | | | |
|---|---|---|---|
| Ber.: | C 32,74 | H 2,29 | F 43,16 |
| Gef.: | C 32,6 | H 2,2 | F 43,1 |

$^1$N-NMR ($CDCl_3$): 1,38 (t, J = 7 Hz, 3H, $CH_3$); 4,43 (q, J = 7 Hz, 2H, $CH_2$);
$^{19}$F-NMR ($CDCl_3$): -75,05 (3F, $CF_3$); -113,73 (2F, $CF_2$);
IR (neat): 5,33 µ (CO); 5,56 µ (-COO-)

Nach diesen eindeutigen Daten kann die in J. Am. Chem. Soc. 75, 3152 (1955) einem Siedepunkt von 130–131°C zugeordnete Verbindung $CF_3COCF_2COOC_2H_5$ diese Verbindung nicht gewesen sein.

Beispiel 14
Perfluor-2-methyl-3-oxa-heptan-6-on-carbonsäuremethylester

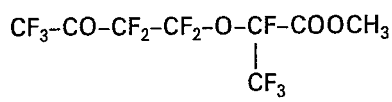

Die Reaktion wird in einem Abzug durchgeführt.

In einem trockenen Kolben mit Magnetrührer, Thermometer, Rückflusskühler und Blasenzähler legt man 70 g (0,15 Mol) 6-Fluorsulfatoperfluor-2-methyl-3-oxaheptansäuremethylester bei -5°C vor. Dann gibt man 2,2 g (0,02 Mol) 1,4 Diazabicyclo/2,2,2/octan zu. Es setzt sofort eine leichte Gasentwicklung ein. Man lässt bei ca. 0°C abreagieren. Der Ansatz rührt eine Stunde bei RT nach und wird anschliessend destilliert. Mit Kp. 131–132°C (745 torr) werden 45 g (82%) Ketoester erhalten.

Analyse:

| | | | |
|---|---|---|---|
| Ber.: | C 25,82 | H 0,81 | F 56,16 |
| Gef.: | C 25,35 | H 0,80 | F 55,90 |

$^1$H-NMR ($CDCl_3$): 3,95 (S)
$^{19}$F-NMR ($CDCl_3$): -75,56 (3F, -CO-$CF_3$); -79,5 (dm, J = 145 Hz, 1F, -O-$CF_2$-); -84,83 (3F, $CF_3$); -88,4 (dm, J = 145 Hz, 1F, -O-$CF_2$); -120,13 (dm, J = 288 Hz, 1F, -CO-$CF_2$-); -123,7 (dm, J = 288 Hz, 1F, -CO-$CF_2$-); -132,5 (1F, CF)
IR (neat): 5,56 μ (C=O)

Beispiel 15
Perfluor-3-oxo-2,4-dimethylpentansäuremethylester

```
     CF3     CF3
      |       |
CF3-CF-CO-CF-COOCH3
```

Die Reaktion wird in einem Abzug durchgeführt.

In einen trockenen Kolben mit Magnetrührer, Thermometer, Vigreux-Kolonne, Kolonnenkopf und Blasenzähler gibt man 0,6 g (0,01 Mol) Kaliumfluorid und 22 g (0,048 Mol) 3-Fluorsulfatoperfluor-2,4-dimethylpentansäuremethylester. Beim Erhitzen des Ansatzes wird ab 100°C Innentemperatur Gasentwicklung beobachtet, die ab 110°C stürmisch wird. Nachdem die Gasentwicklung abgeklungen ist, wird der Ansatz destilliert. Mit Kp. 128°C (748 mm) werden 14,4 g (84%) Perfluor-3-oxo-2,4-dimethylpentansäuremethylester erhalten.

Analyse:

| | | | |
|---|---|---|---|
| Ber.: | C 26,98 | H 0,85 | F 58,69 |
| Gef.: | C 26,9 | H 0,9 | F 58,8 |

$^1$H-NMR ($CDCl_3$): 3,98 (S)
$^{19}$F-NMR ($CDCl_3$): -73,27 (3F, $CF_3$); -73,61 (3F, $CF_3$); -75,19 (3F, $CF_3$); -175,93 (1F, -CO-CF-CO-); -187,62 (1F, CF)
IR (neat): 5,51 μ (CO), 5,60 μ (CO)

Beispiel 16
Perfluor-3-oxo-2,4-dimethylpentansäurefluorid

```
     CF3     CF3
      |       |
CF3-CF-CO-CF-COF
```

Die Reaktion wird in einem Abzug durchgeführt.

In einem trockenen Kolben mit Magnetrührer, Thermometer, Tropftrichter, Rückflusskühler und Blasenzähler legt man bei RT 0,6 g (0,01 Mol) trockenes, fein-pulverisiertes Kaliumfluorid vor. Nach dem Zutropfen von 30 g (0,067 Mol) 3-Fluorsulfatoperfluor-2,4-dimethylpentansäurefluorid wird der Ansatz erhitzt. Ab ca. 100°C Innentemperatur tritt Gasentwicklung auf. Nachdem die Gasentwicklung beendet ist, wird der Ansatz über eine Füllkörperkolonne destilliert. Mit Kp. 72-74°C (760 mm) werden 14,2 g (61%) Perfluor-3-oxo-2,4-dimethylpentansäurefluorid erhalten.

$^{19}$F-NMR ($CDCl_3$): +32,09 (1F, COF); -73,28 (3F, $CF_3$); -73,84 (3F, $CF_3$); -74,51 (3F, $CF_3$); -175,25 (1F, CF); -187,49 (1F, Isopropyl-CF)
IR (Gasspektrum): 5,24 μ und 5,32 μ (COF); 5,58 μ (CO)

Beispiel 17
Perfluor-(2-methyl-hexandion-3,5)

```
CF3-C-CF2-C-CF(CF3)2
    ||    ||
    O     O
```

In einem 200 ml Dreihalskolben mit Tropftrichter, Thermometer, Magnetrührer und Raschig-Ringkolonne mit Destillationskopf für Tieftemperaturdestillation werden 4 g CF vorgelegt und im Vakuum ausgeheizt. Im Laufe von 1 Stunde werden 68,7 g (0,154 Mol) Perfluor(5-fluorsulfato-2-methylhexan-3-on), $(CF_3)_2CF$-CO-$CF_2$-$CF(CF_3)OSO_2F$, bei RT zugetropft. Dabei erwärmt sich das Reaktionsgemisch langsam auf 40°C. Durch fraktionierte Destillation lassen sich neben 15 g $SO_2F_2$ (= 95% d.Th.) 36 g (0,105 Mol) Perfluor-(2-methylhexandion-3,5) vom Siedepunkt 76°C (745 mm) isolieren. Ausbeute 68% d.Th.

```
CF3-C-CF2-C-CF(CF3)2
    ||    ||
    O     O
```

$C_7F_{12}O_2$ MG: 344 Ber.: C 24,4 F 66,3
Gef.: C 23,7 F 65,9
$^{19}$F-NMR ($C_6D_6$): -74,0 (6F, $CF_3$); -75,2 (3F, $CF_3$); -113,2 (2F, $CF_2$); -191,2 (1F, CF);
IR (neat): 5,55 μ (C=O)

**Patentansprüche**

1. Verfahren zur Herstellung von Polyfluorcarbonylverbindungen der Formel V

```
Rf-C-R'f-[CF2-O-CF]n-COR          (V)
   ||             |
   O              CF3
```

worin R = F, Cl oder Perfluoralkyl mit 1-10, vorzugsweise 1-8, insbesondere 1-3 C-Atomen, oder OR‴ mit
R‴ = Alkyl, Aryl oder Aralkyl mit vorzugsweise bis zu 10 C-Atomen, insbesondere $CH_3$ oder $C_2H_5$,
$R_f$ = Perfluoralkyl mit 1-10, vorzugsweise 1-8, insbesondere 1-3 C-Atomen,
$R'_f$ = Perfluoralkylen mit 1-10, vorzugsweise 1-6, insbesondere 1-2 C-Atomen, und
n = 0-10, vorzugsweise 0-4, insbesondere 0 oder 1 bedeuten,
dadurch gekennzeichnet, dass man

a) Polyfluorcarbonylverbindungen mit noch einem sec. H-Atom der Formel I

```
Rf-CF-R'f-[CF2-O-CF]n-COR"        (I)
   |              |
   H              CF3
```

worin $R_f$, und $R'_f$ und n die gleiche Bedeutung wie in Formel V besitzen und
R″ = F, Cl oder Perfluoralkyl mit 1-10, vorzugsweise 1-8, insbesondere 1-3 C-Atomen, ist,
in einem Elektrolyten aus Fluorsulfonsäure und einem Alkalifluorsulfonat unter Verwendung von Platin oder Metallen der Platingruppe (Os, Ir, Pt) und/oder glasartigem Kohlenstoff als Anodenmaterialien und von üblichen, jedoch unter den Elektrolysebedingungen stabilen Kathodenmaterialien zu den Fluorsulfatoverbindungen der Formel II

$R_f$-CF-$R'_f$-[$CF_2$-O-CF]$_n$-COR″ (II)
|  |
$OSO_2F$  $CF_3$

worin $R_f$, $R'_f$, R″ und n die gleiche Bedeutung wie in Formel I besitzen,
elektrolysiert,

b) die Fluorsulfatoverbindungen der Formel II gegebenenfalls - wenn R″ = F oder Cl - mit einer organischen Hydroxylverbindung der Formel III

R‴OH (III)

worin R‴ die bei Formel V genannte Bedeutung besitzt, verestert, und

c) die nach den Reaktionsstufen a und b resultierenden Verbindungen der Formel IV

$R_f$-CF-$R'_f$-[$CF_2$-O-CF]$_n$-COR (IV)
|  |
$OSO_2F$  $CF_3$

worin R, $R_f$, $R'_f$ und n die gleiche Bedeutung wie in Formel V besitzen, in Gegenwart katalytischer Mengen von Alkalifluoriden und/oder aprotischen N-Basen zu Verbindungen der Formel V zersetzt.

2. Perfluorcarbonylverbindungen der Formel IV

$R_f$-CF-$R'_f$-[$CF_2$-O-CF]$_n$-COR (IV)
|  |
$OSO_2F$  $CF_3$

worin $R_f$ = Perfluoralkyl mit 1-10, vorzugsweise 1-8, insbesondere 1-3 C-Atomen,
$R'_f$ = Perfluoralkylen mit 1-10, vorzugsweise 1-6, insbesondere 1-2 C-Atomen,
R = F, Cl, Perfluoralkyl mit 1-10, vorzugsweise 1-8, insbesondere 1-3 C-Atomen oder
-OR‴ (R‴ = Alkyl, Aryl oder Aralkyl mit vorzugsweise bis zu 10 C-Atomen, insbesondere $CH_3$ oder $C_2H_5$), und
n = 0-10, vorzugsweise 0-4, insbesondere 0 oder 1
bedeuten.

**Claims**

1. A process for the preparation of polyfluorocarbonyl compounds of the formula V

$R_f$-C-$R'_f$-[$CF_2$-O-CF]$_n$-COR (V)
||  |
O  $CF_3$

in which
R = F, Cl or perfluoroalkyl with 1-10, preferably 1-8 and in particular 1-3 C-atoms, or OR‴ with R‴ = alkyl, aryl or aralkyl with preferably up to 10 C-atoms, in particular $CH_3$ or $C_2H_5$,
$R_f$ = perfluoroalkyl with 1-10, preferably 1-8 and in particular 1-3 C-atoms,
$R'_f$ = perfluoroalkylene with 1-10, preferably 1-6 and in particular 1-2 C-atoms, and
n = 0-10, preferably 0-4 and in particular 0 or 1,
characterized in

a) electrolyzing perfluorocarbonyl compounds also having a secondary H atom, of the formula I

$R_f$-CF-$R'_f$-[$CF_2$-O-CF]$_n$-COR″ (I)
|  |
H  $CF_3$

in which $R_f$, $R'_f$ and n have the same meaning as in formula V and R″ = F, Cl or perfluoroalkyl with 1-10, preferably 1-8 and in particular 1-3 C-atoms in an electrolyte consisting of fluorosulfonic acid and an alkali metal fluorosulfonate, using platinum or metals of the platinum group (Os, Ir or Pt) and/or glassy carbon as the anode materials and cathode materials which are customary but are stable under the electrolysis conditions, to give the fluorosulfato compounds of the formula II

$R_f$-CF-$R'_f$-[$CF_2$-O-CF]$_n$-COR″ (II)
|  |
$OSO_2F$  $CF_3$

in which $R_f$, $R'_f$, R″ and n have the same meaning as in formula I,

b) if necessary - if R″ = F or Cl - esterifying the fluorosulfato compounds of the formula II with an organic hydroxy compound of the formula III

R‴OH (III)

in which R‴ has the same meaning as in formula V and

c) decomposing the compounds resulting from reaction stages a and b, of the formula IV

$R_f$-CF-$R'_f$-[$CF_2$-O-CF]$_n$-COR (IV)
|  |
$OSO_2F$  $CF_3$

in which R, $R_f$, $R'_f$ and n have the same meaning as in formula V in the presence of catalytic amounts of alkali metal fluorides and/or aprotic N-bases to give the compounds of the formula V.

2. Perfluorocarbonyl compounds of the formula IV

$R_f$-CF-$R'_f$-[$CF_2$-O-CF]$_n$-COR (IV)
|  |
$OSO_2F$  $CF_3$

in which $R_f$ = perfluoroalkyl with 1-10, preferably 1-8 and in particular 1-3 C-atoms, $R'_f$ = perfluoroalkylene with 1-10, preferably 1-6 and in particular 1-2 C-atoms, R = F, Cl perfluoroalkyl with 1-10, preferably 1-8, in particular 1-3 C-atoms or -OR‴ (R‴ = alkyl, aryl or aralkyl with preferably up to 10 C-atoms, in particular $CH_3$ or $C_2H_5$), and n = 0 to 10, preferably 0 to 4 and in particular 0 or 1.

**Revendications**

1. Procédé de préparation de composés polyfluorocarbonylés de formule V:

$$\begin{array}{lll} R_f\text{-C-}R'_f\text{-}[CF_2\text{-O-}CF]_n\text{-COR} & & \text{(V)} \\ \quad \| \qquad\qquad\quad | & & \\ \quad O \qquad\qquad\; CF_3 & & \end{array}$$

[dans laquelle R représente F, Cl ou un groupe perfluoroalkyle ayant 1 à 10, avantageusement 1 à 8, notamment 1 à 3 atomes de carbone, ou OR‴, R‴ étant un groupe alkyle, aryle ou aralkyle ayant avantageusement jusqu'à 10 atomes de carbone, notamment $CH_3$ ou $C_2H_5$;
$R_f$ est un groupe perfluoro alkyle ayant 1 à 10, avantageusement 1 à 8, notamment 1 à 3 atomes de carbone;
$R'_f$ est un groupe perfluoralkylène ayant 1 à 10, avantageusement 1 à 6, notamment 1 ou 2 atomes de carbone, et
n vaut 0 à 10, avantageusement 0 à 4, notamment 0 ou 1]
caractérisé en ce que:

a) on électrolyse des composés polyfluorocarbonylés comportant encore un atome de H secondaire, de formule I:

$$\begin{array}{lll} R_f\text{-CF-}R'_f\text{-}[CF_2\text{-O-}CF]_n\text{-COR''} & & \text{(I)} \\ \quad | \qquad\qquad\quad | & & \\ \quad H \qquad\qquad\; CF_3 & & \end{array}$$

(dans laquelle $R_f$, $R'_f$ et n ont le même sens que pour la formule V, et
R″ représente F, Cl ou un groupe perfluoroalkyle ayant 1 à 10, avantageusement 1 à 8, notamment 1 à 3 atomes de carbone), dans un électrolyte formé d'acide fluorosulfonique et d'un fluorosulfonate alcalin en utilisant du platine ou des métaux de la famille du platine (Os, Ir, Pt) et/ou du carbone vitreux comme matières d'anode et des matières usuelles, mais cependant stables dans les conditions de l'électrolyse, pour la cathode pour obtenir les composés fluorosulfato de formule II:

$$\begin{array}{lll} R_f\text{-CF-}R'_f\text{-}[CF_2\text{-O-}CF]_n\text{-COR''} & & \text{(II)} \\ \quad | \qquad\qquad\quad | & & \\ \quad OSO_2F \qquad CF_3 & & \end{array}$$

(dans laquelle $R_f$, $R'_f$, R″ et n ont le même sens que pour la formule I),

b) on estérifie éventuellement, lorsque R″ représente F ou Cl, les composés fluorosulfato de formule II avec un composé organique hydroxylé de formule III:

$$R'''OH \qquad \text{(III)}$$

(dans laquelle R‴ a le sens indiqué pour la formule V), et

c) on soumet les composés obtenus après les étapes de réaction a) et b), de formule IV:

$$\begin{array}{lll} R_f\text{-CF-}R'_f\text{-}[CF_2\text{-O-}CF]_n\text{-COR} & & \text{(IV)} \\ \quad | \qquad\qquad\quad | & & \\ \quad OSO_2F \qquad CF_3 & & \end{array}$$

(dans laquelle R, $R_f$, $R'_f$ et n ont le même sens que pour la formule V) à une décomposition en présence de quantités catalytiques de fluorures alcalins et/ou de bases azotées aprotiques pour obtenir les composés de formule V.

2. Composés perfluorocarbonylés de formule IV:

$$\begin{array}{lll} R_f\text{-CF-}R'_f\text{-}[CF_2\text{-O-}CF]_n\text{-COR} & & \text{(IV)} \\ \quad | \qquad\qquad\quad | & & \\ \quad OSO_2F \qquad CF_3 & & \end{array}$$

(dans laquelle $R_f$ est un groupe perfluoroalkyle ayant 1 à 10, avantageusement 1 à 8, notamment 1 à 3 atomes de carbone,
$R'_f$ est un groupe perfluoralkylène ayant 1 à 10, avantageusement 1 à 6, notamment 1 ou 2 atomes de carbone,
R représente F, Cl, un groupe perfluoroalkyle ayant 1 à 10, avantageusement 1 à 8, notamment 1 à 3 atomes de carbone, ou -OR‴ (R‴ étant un groupe alkyle, aryle ou aralkyle ayant avantageusement jusqu'à 10 atomes de carbone, notamment $CH_3$ ou $C_2H_5$), et
n vaut 0 à 10, avantageusement 0 à 4, notamment 0 ou 1).